# EUROPEAN PATENT APPLICATION

(11) **EP 4 534 112 A1**
(43) Date of publication of application: **09.04.2025**
(21) Application number: 24177858.8
(22) Date of filing: 24.05.2024
(51) Int. Cl.: A61L 2/20, D06M 11/34

(54) **DEVICE AND METHOD FOR DISINFECTION OF CONTAMINATED CLOTHING PRODUCTS**

(30) Priority: 06.10.2023 RO 202300556
(71) Applicant: Universitatea Tehnica "Gheorghe Asachi" Din Iasi, 700050 Iasi (RO)
(72) Inventor: Bodoga, Alexandra, Iasi (RO); NISTORAC, Andreea, Iasi (RO); LOGHIN, Maria-Carmen, Iasi (RO)
(74) Representative: Dobrescu, Teodora Valentina

(57) **Abstract**

The present invention relates to a device for disinfection of clothing products contaminated with pathogens comprising a gas impermeable enclosure equipped with a sealing system wherein the gas impermeable enclosure has a one-way mechanical valve connectable to a vacuum pump, said mechanical valve being provided with a closing cap; and wherein the gas impermeable enclosure further comprises an input connectable to an ozone generator.

The invention also discloses a method for disinfection of clothing products contaminated with pathogens with the device of the invention comprising the step of introducing the products to be disinfection into the gas impermeable enclosure, vacuuming the air, and injecting ozone at a certain concentration for a specific time.

## Description

### Field of Invention

The invention relates to a device for disinfection of clothing products contaminated with pathogens. Specifically, the invention relates to a device and a method for disinfection of clothing products contaminated with pathogens using ozone.

### State of the art

The rapid change in consumer trends, namely the "fast fashion" movement, has led to an increase in the consumption of shoes and clothing, respectively, and a decrease in the lifespan of products. In the current stage, there is an increase in production and implicitly in the waste resulting from the manufacturing process as well as in products that, although they are functional, end up in landfills, being morally outdated. According to the report on the Effectiveness of waste management policies in the European Union (EEA report - 2009) 79.2% of footwear products end up in landfills, 19.8% are incinerated and only 1% are recycled. The accumulation of textile waste in landfills contributes to environmental pollution and resource depletion.

For this reason, special importance must be given to the methods of waste minimization and valorisation, and their correct management from a sustainable point of view. The technological solutions for waste management, namely the "3R" (reuse, reduction, recycling), currently available, favourably influence the impact of used footwear and clothing products on the environment.

However, it was found worldwide that the issue of disinfection before or during the recycling process is less addressed in the case of used products that may carry pathogens. In this sense, new actions, and research studies regarding reintroducing the product or its components into the economic circuit under biological safety conditions are required.

Before being recycled, these products must be disinfected to avoid the spread of bacteria and other harmful substances. The presence of pathogens in textile waste is a cause for concern, primarily because of the potential threats they pose to public health. The current state of textile waste highlights the need for effective cleaning strategies.

Cleaning textile waste presents several challenges. These include the presence of contaminants such as dyes, chemicals, and pathogens, as well as variability in fabric composition and structural integrity.

Traditional cleaning methods, such as water-based washing, may not be suitable for all types of textile waste and may require significant energy and water consumption. Textiles are essential materials in many industries, including healthcare and food service, where cleanliness is essential to prevent the spread of infection. However, they can easily be contaminated with bacteria, viruses, or other harmful microorganisms that can cause disease.

Several methods of textile disinfection can be discussed, including chemical, thermal, and physical techniques and their effectiveness in eliminating microorganisms. However, most of these disinfection methods result in harmful chemical residues that need to be neutralized or the costs associated with using them are too high and thus become economically inefficient.

This underlines the imperative need for the implementation of robust mitigation strategies and an increased focus on research efforts aimed at understanding the complicated dynamics of pathogen transmission. At the same time, these efforts aim to develop effective prevention and control measures. By diligently pursuing these goals, potential health hazards arising from textile waste can be effectively mitigated, ultimately fostering a safer and more sustainable environment.

Therefore, it is necessary to implement new and effective disinfection methods to ensure the safety and health of users.

**The purpose of the invention** is to provide an effective disinfection method and a device for disinfection of contaminated clothing products with pathogens that will remove the drawbacks mentioned herein or other drawbacks existing in the state of the art. Also, another purpose is to find new eco-friendly and economical ways of disinfection methods that are easy to use, with high disinfection rates.

**The purpose of the invention is achieved** by providing the device for disinfection comprising a gas impermeable enclosure equipped with a sealing system wherein the gas impermeable enclosure has a one-way mechanical valve connectable to a vacuum pump, said mechanical valve being provided with a closing cap and wherein the gas impermeable enclosure further comprises an input connectable to an ozone generator.

Another purpose of the invention is achieved by providing the method for disinfection of the contaminated clothing products using the device of the invention comprising the step of introducing the products to be disinfection into the gas impermeable enclosure, vacuuming the air and injecting ozone at a certain concentration for a specific time.

### Brief description of the invention

The present invention provides a device for the disinfection of clothing products contaminated with pathogens comprising a gas impermeable enclosure equipped with a sealing system wherein the gas impermeable enclosure has a one-way mechanical valve connectable to a vacuum pump, said mechanical valve being provided with a closing cap; and wherein the gas impermeable enclosure further comprises an input connectable to an ozone generator.

The invention also provides a method for disinfection of clothing products contaminated with pathogens with the device of the invention comprising the step of introducing the products to be disinfected into the gas impermeable enclosure, vacuuming the air, and injecting ozone at a certain concentration for a specific time.

### Definitions

Disinfection is the process of removing micro-organisms, including potentially pathogenic ones, from the surfaces of inanimate objects.

Polypropylene (PP) is a thermoplastic polymer used in a range of domestic and industrial applications. It is produced via chain-growth polymerization from the monomer propylene.

Gas impermeable enclosure refers to a structure or container that is sufficiently airtight to allow for the near-total removal of air from the interior and to prevent the ingress of air or other gases into the enclosure, ensuring the maintenance of a controlled environment without external gas contamination.

Plant-based materials refers to materials and products that are partially or wholly derived from plants or other renewable agricultural, aquatic, or forestry inputs. Paptic^{®} material is a mixture of wooden fibres and cellulose.

Polyethylene and polyamide mixture (nylon) is a semi-crystalline thermoplastic with low density and high thermal stability.

Polyester-spandex is a blend of two synthetic fibres, polyester, and spandex. Is a synthetic fibre known for its exceptional elasticity.

Sustainable bioplastics are typically plastics manufactured from bio-based polymers, made from natural resources.

Low bulk density refers to a fabric characteristic where the material has a relatively low weight per unit volume and has a structure that incorporates a significant amount of air and with values that fall between 50 to 100 kg/m³.

Medium bulk density refers to a fabric characteristic where the material has a moderate weight per unit volume, providing a balance between heaviness and lightness. These fabrics have a denser structure compared to low bulk-density fabrics but still, maintain some air within the fibres. Values for medium bulk density fabrics fall between 100 to 250 kg/m³.

High bulk density refers to a fabric characteristic where the material has a relatively high weight per unit volume, indicating a dense and compact structure with minimal air content. These fabrics are generally heavier and less airy, providing substantial weight and durability. Values for high bulk density fabrics exceed 250 kg/m³.

Heavy fabrics are textiles characterized by a high weight per unit area, typically exceeding 300 kg/m².

Denim is a firm durable twilled usually cotton fabric woven with coloured warp and white filling thread.

Vacuum enclosure - almost all the air was removed from the enclosure (about 99.7%).

### Ozone O₃

### Brief description of the drawing

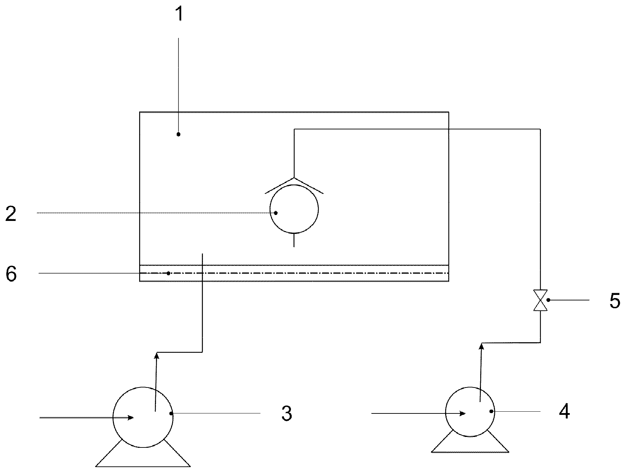

Figure 1 is a schematic representation of the device of the invention.

### Description of the invention

The invention will be described in more detail below.

In a first aspect, the invention provides a device for the disinfection of clothing products contaminated with pathogens comprising a gas-impermeable enclosure (1) equipped with a sealing system (6) characterized by the fact that the gas-impermeable enclosure (1) has a one-way mechanical valve (2) connectable to a vacuum pump (4) wherein the mechanical valve is provided with a closing cap (5); and by that, the gas impermeable enclosure (1) further comprises an input connectable to an ozone generator (3).

In a preferred embodiment, the invention provides the device for disinfection of clothing products contaminated with pathogens wherein the gas impermeable enclosure (1) is made of plastic material.

In one embodiment, the plastic material is selected from, but not limited to, polypropylene (PP), polyethylene and polyamide mixture (nylon), polyester-spandex, sustainable bioplastics, and other total or partial recycled plastic materials.

In an embodiment preferred in particular, the gas-impermeable enclosure is made of PP. The preferred embodiment is PP because it is easy to use, has a low cost, and is very durable as it can be used at multiple cycles of disinfection until the mechanical integrity of the material is affected.

However, any person skilled in the art will determine when the mechanical integrity of the gas impermeable enclosure is affected and will use a new gas impermeable enclosure.

In another embodiment, the gas-impermeable enclosure (1) is a plastic bag.

In another embodiment, the invention provides the device for the disinfection of clothing products contaminated with pathogens wherein the gas impermeable enclosure (1) is made of biodegradable materials (plant-based materials) selected from, but not limited to, cotton, hemp, or mixture thereof.

In a further embodiment, the invention provides the device for disinfection of clothing products contaminated with pathogens wherein the gas impermeable enclosure (1) is made of Paptic^{®} materials which is a mixture of wooden fibres and cellulose.

In yet another embodiment, the invention provides the device for the disinfection of clothing products contaminated with pathogens wherein the gas impermeable enclosure (1) is made of a combination of bioplastics and plant-based materials.

In another embodiment of this invention, the device for disinfection of clothing products contaminated with pathogens, the pathogen is selected from, but not limited to, bacteria, viruses, and fungi.

In a particular embodiment, the bacteria is selected from, but not limited to, *Escherichia coli, Staphylococcus aureus, Klebsiella pneumoniae,* and *Pseudomonas aeruginosa.* In another embodiment, the fungi are selected from, but not limited to, Aspergillus, *Candida,* and *Trichophyton.* In a further embodiment, the viruses are selected from but are not limited to, *influenta viruses* and *noroviruses.*

An embodiment more preferred is the device for disinfection of clothing products contaminated with pathogens wherein the pathogen is *Escherichia coli* and *Candida Albicans,* especially.

In a second aspect, the invention provides a method for disinfection of clothing products contaminated with pathogens with the device of the invention comprising the following steps:
a. introducing the clothing products in the gas impermeable enclosure (1);
b. vacuuming the air from the gas impermeable enclosure (1) with a vacuum pump;
c. injecting ozone into the gas impermeable enclosure (1) at a flow rate of between 78.33 milligrams O₃/hour and 88.33 milligrams O₃/hour, preferably at the flow rate of 83.33 milligrams O₃/hour wherein the flow rate of the O₃ is maintained constant for some time of between 30 to 60 minutes.

The success of the disinfection process disclosed in this invention depends on the time of the ozone injection, on constant flow rate of the ozone, and of the fact that the gas impermeable enclosure is vacuumed. The synergic effect of these 3 features results in a disinfection rate of at least 99.7 % for the 30-minute process and 99.9 % for the 60-minute process.

Also, the flow rate of the ozone (O₃) must be maintained constant throughout the process of disinfection.

In a further embodiment, the dimensions of the gas impermeable enclosure (1) are between 38 cm x 42 cm to 110 cm x 130 cm, having a volume of between 4500 cm³ to 50.000 cm³. It is possible to use bigger gas impermeable enclosures, but it becomes very difficult to manipulate them and the process becomes more difficult to perform.

It is also important that the gas impermeable enclosure (1) has a thickness of between 100 to 200 micrometres, preferably between 130 to 180 micrometres, and most preferably between 150 to 160 micrometres.

Also, this thickness is chosen between the intervals mentioned above because the gas-impermeable enclosure is sufficiently elastic and resistant to be used in multiple disinfection cycles.

In another embodiment, the method of the invention uses a gas impermeable enclosure (1) made of plastic material selected from PP, polyethylene, polyamide mixture (nylon), polyester-spandex, and sustainable bioplastics.

In the most preferred embodiment, the method of the invention uses a gas-impermeable enclosure (1) made of PP.

In a further embodiment, the method of the invention uses a gas impermeable enclosure (1) is made of biodegradable materials (plant-based materials) selected from, but limited to, cotton, hemp, or mixture thereof.

Also, if we consider the maximum volume of the gas impermeable enclosure of 50.000 cm³ and take into consideration the density of the clothing products, the device, and the method disclosed in the present invention can be used for the disinfection of:
- 1-17 kg of materials and a mixture of materials having low bulk density, for example, cotton and elastane;
- 1-25 kg of materials and a mixture of materials having medium bulk density, for example, polyester, wool, and materials with hydrophobic treatments;
- 1-35 kg of materials and a mixture of materials having high bulk density, for example - heavy fabrics and denim.

**Table 1. Textile fabric parameters: thickness h (mm), area density ρₛ(g.m⁻²), bulk density ρᵥ(kg.m⁻³) and material composition.**

| Sample n. | Material composition | Materials properties | | |
|---|---|---|---|---|
| | | *ρₛ* (g.m⁻²) | *h* (mm) | *ρᵥ* (kg.m⁻³) |
| 1 | 65/35 % Polyester/cotton hydrophobic treatment | 182 | 0.36 | 506 |
| 2 | 44/53/3 % Wool/polyester/elastane | 212 | 0.47 | 451 |
| 3 | 43/54/3 % Wool/polyester/elastane | 187 | 0.43 | 435 |
| 4 | 96/4 % cotton/elastane | 180 | 0.52 | 346 |
| 5 | 100% cotton | 200 | 0.47 | 426 |
| 6 | 100% cotton | 245 | 0.58 | 422 |
| 7 | 100% cotton | 300 | 0.59 | 509 |

It is obvious for a specialist in the art how to calculate the ratios between the volume of the gas impermeable enclosure (1) and the volume/mass of the clothing products to be disinfection having in mind the teachings disclosed in this invention.

A particularly preferred embodiment is a method for disinfection of clothing products contaminated with pathogens wherein vacuuming the air from the gas impermeable enclosure (1) is made at a pressure of between 7.85 kPa and 85 kPa, preferably 47 kPa.

The pump will remove about 99.8% of the air contained in the gas-impermeable enclosure (1) and thus remove all the air between the clothing products. Thus, the process is efficient even if the clothing products are stacked between them or if wrinkles are formed.

The invention disclosed herein can be used for disinfection for all textile materials and other aerated materials of low density (sponges, lining fillings, pillows, jackets, insulating materials such as mineral wool), small medical equipment with closed components (ophthalmoscope, dermoscope, pulse oximeter, blood pressure monitor, heart rate monitor) contact electronics (audio headphones, body aesthetic equipment).

### Specific Embodiment

In the following, are provided two specific embodiments that should not be considered limiting the invention in any way.

In a first specific embodiment, the invention provides a device for the disinfection of clothing products contaminated with pathogens comprising a gas-impermeable enclosure (1) made of PP equipped with a sealing system (6) wherein the gas-impermeable enclosure (1) has a one-way mechanical valve (2) connectable to a vacuum pump (4) wherein the mechanical valve is provided with a closing cap (5); and wherein the PP gas impermeable enclosure (1) further comprises an input connectable to an ozone generator (3).

For this specific embodiment, the gas impermeable enclosure is a plastic bag of PP and has the dimensions of 38 cm x 42 cm (the volume is about 4500 cm³). This gas-impermeable enclosure can be used for multiple disinfection cycles until the mechanical integrity of the plastic bag is affected. Any person skilled in the art will determine when the mechanical integrity of the gas impermeable enclosure is affected and will use a new bag.

It was tested for clothing products contaminated with *Escherichia coli* and/or *Candida Albicans.*

Into a second specific embodiment, this invention provides a method for disinfection of clothing products contaminated with pathogens using the above-mentioned device comprising the following steps:
a. introducing the clothing products in the gas impermeable enclosure (1) of plastic material made of PP with the dimensions of 38 cm x 42 cm (the volume is about 4500 cm³). In the conducted experiments samples made from cotton (low to medium bulk density), polyester (high bulk density), spandex (high bulk density), and pig leather lining (medium to heavy bulk density) were placed between layers of textile materials to simulate the presence of biological contaminants within a cluster of materials;
b. vacuuming the air from the gas impermeable enclosure (1) with a vacuum pump at a pressure of 47,41 kPa;
c. injecting ozone into the PP gas impermeable enclosure (1) at a flow rate of 83,33 milligrams O₃/hour, for 30 minutes (for the first sets of samples) and respectively 60 minutes (for the second set of samples)

Any vacuum pump and any ozone generator suitable for this invention can be used.

### Experimental part

In this part, it was determined the efficiency of the disinfection process using the device of the invention.

Experiments were carried out in which a pathogenic bacterial strain, namely *Escherichia coli,* isolated from contaminated water samples, and a yeast strain - *Candida albicans,* isolated from the skin were used. There were used clothing products made of cotton, elastane, polyester, and pig leather lining in the experiments.

The disinfection rates as shown in the tables below are all above 99%.

The lowest rates for 30-minute ozone treatment are 99.74% for *E*. *coli* contamination recorded for pigskin lining samples and 99.77% for *C. albicans* contamination recorded for polyester-spandex blend samples.

For the 60-minute ozone treatment, all samples reached a disinfection degree of 99.99% for both strains, except for the polyester-spandex blend sample contaminated with *C. albicans,* which achieved a disinfection degree of 99, 98%. Vacuum ozone disinfection rates of over 99% have been demonstrated for heavily used textile samples against both bacterial and fungal species.

Efficacy against viruses has been demonstrated in pandemics, particularly SARS-like viruses.

In the below table, a comparison between the initial degree of contamination of textile samples with strains of *E. coli* and *C. Albicans* and disinfection results using the method of the invention for 30 and 60 minutes is given.

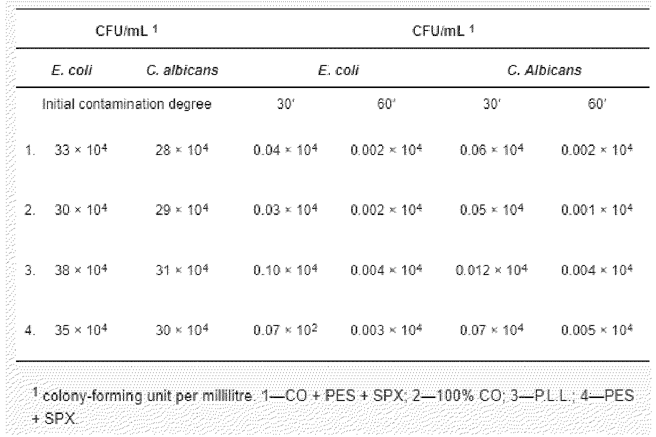

The degree of disinfection of clothing products after treatment of 30 and 60 minutes using the method of the present invention initially contaminated with *E*. *coli* and *C*. *albicans.*

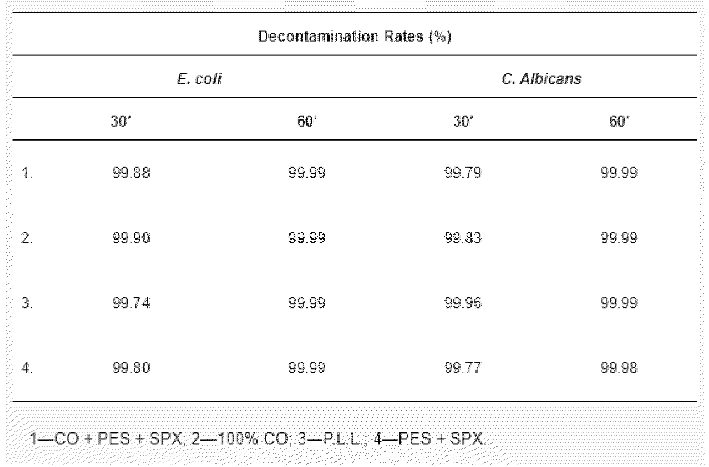

### Advantages of the invention

The present invention provides a simple device for the disinfection of clothing products contaminated with pathogens comprising a gas-impermeable enclosure made of plastic material equipped with a sealing system wherein the gas-impermeable enclosure has a one-way mechanical valve connectable to a vacuum pump, said mechanical valve is provided with a closing cap; and wherein the gas impermeable enclosure further comprises an input connectable to an ozone generator. The invention also provides a method for disinfection of clothing products contaminated with pathogens with the device of the invention comprising the step of introducing the products to be disinfected into the gas impermeable enclosure, vacuuming the air, and injecting ozone at a certain flow rate for a specific time.

The advantages of the device and method provided in this invention are:
- The disinfection process is a dry process being made with ozone gas;
- the disinfectant, namely the ozone (O₃) gas, is consummated without leaving any residues that need to be neutralized later on;
- by vacuuming and removing the air from the gas-impermeable enclosure, between the textile products, as well as inside the structures from which the textile products are made, it allows ozone to act effectively on pathogens regardless of their positioning on the product to be disinfected;
- the quantity of ozone is minimal because the removal of the air from the gas-impermeable enclosure results in the direct action of the ozone on the clothing/textile to be disinfected; also, the ozone has a specific flow rate and a long time of action until it is consumed;
- there is the possibility to disinfect different quantities of clothing according to the necessity and to the volume of the gas-impermeable enclosure;
- simultaneously with the disinfection, their odors are also carried out.
- the possibility of using the device and the method from the invention by vacuuming and introducing ozone into the gas-impermeable enclosure sealing it and repeating this process with another gas-impermeable enclosure loaded with a new batch of clothing products to be disinfected;
- the disinfection method and device are simple, and easy to use, with very high disinfection rates and low cost;
- also, it does not use any chemical agents that need to be deactivated/neutralized after the ending of the process;
- it has reproducible results with very high disinfection rates;

## Claims

1. Device for disinfection of clothing products contaminated with pathogens comprising a gas impermeable enclosure (1) equipped with a sealing system (6) **characterized by** the fact that the gas impermeable enclosure (1) has a one-way mechanical valve (2) connectable to a vacuum pump (4) wherein the mechanical valve is provided with a closing cap (5);
**and by that,** the gas impermeable enclosure (1) further comprises an input connectable to an ozone generator (3).

2. Device for disinfection of clothing products contaminated with pathogens according of claim 1 wherein the gas impermeable enclosure (1) is made of plastic material.

3. Device for disinfection of clothing products contaminated with pathogens according to claim 2 wherein the plastic material is selected from PP, polyethylene, polyamide mixture (nylon), polyester-spandex, sustainable bioplastics, preferably PP.

4. Device for disinfection of clothing products contaminated with pathogens according to claims 2-3 wherein the gas impermeable enclosure (1) is a plastic bag.

5. Device for disinfection products contaminated with pathogens according to claim 1 wherein the gas impermeable enclosure (1) is made of biodegradable materials (plant-based materials) selected from cotton, hemp, or a mixture thereof.

6. Device for disinfection of clothing products contaminated with pathogens according to any of the preceding claims wherein the pathogen is selected from bacteria, viruses, and fungi.

7. Device for disinfection of clothing products contaminated with pathogens according to any of the preceding claims wherein the pathogen is *Escherichia coli* and *Candida Albicans.*

8. Method for disinfection of clothing products contaminated with pathogens with the device of any of the previous claims comprising the following steps:
a. introducing the clothing products in the gas impermeable enclosure (1);
b. vacuuming the air from the gas impermeable enclosure (1) with a vacuum pump;
c. injecting ozone into the gas impermeable enclosure (1) at a flow rate of between 78.33 milligrams O₃/hour and 88.33 milligrams O₃/hour, preferably at a flow rate of 83.33 milligrams O₃/hour
wherein the flow rate of the O₃ is maintained constant for a time of between 30 to 60 minutes.

9. Method for disinfection of clothing products contaminated with pathogens according to claims 8 wherein the gas impermeable enclosure (1) is made of plastic material selected from PP, polyethylene, and polyamide mixture (nylon), polyester-spandex, sustainable bioplastics, preferably PP.

10. Method for disinfection of clothing products contaminated with pathogens according to claim 8 wherein the gas impermeable enclosure (1) is made of biodegradable materials (plant-based materials) selected from cotton, hemp, or a mixture thereof.

11. Method for disinfection of clothing products contaminated with pathogens according to claims 8-10 wherein the thickness of the gas impermeable enclosure (1) is between 100 to 200 micrometres, preferably between 130 up to 180 micrometres, most preferably between 150 to 160 micrometres.

12. Method for disinfection of clothing products contaminated with pathogens according to claims 8-11 wherein the gas impermeable enclosure (1) has a dimension of 38 cm x 42 cm (the volume is about 4500cm³);

13. Method for disinfection of clothing products contaminated with pathogens according to claims 8-12 wherein vacuuming the air from the gas impermeable enclosure (1) is made at a pressure of between 7.85kPa to 85kPa, preferably 47kPa.
